# EUROPEAN PATENT SPECIFICATION

(11) **EP 1 824 492 B1**
(45) Date of publication and mention of the grant of the patent: **10.07.2013**
(21) Application number: 05854982.5
(22) Date of filing: 16.12.2005
(51) Int. Cl.: A61K 31/517, A61P 35/04

(54) **Lapatinib for treating breast cancer brain metastases**
Lapatinib zur Behandlung von Brustkrebs-Hirnmetastasen
Lapatinib pour le traitement de métastases cérébrales du cancer du sein

(30) Priority: 17.12.2004 US 637052 P
(43) Date of publication of application: 29.08.2007
(73) Proprietor: SmithKline Beecham (Cork) Limited, Carrigaline, County Cork (IE)
(72) Inventor: RUBIN, Stephen, Collegeville, PA 19426 (US)
(74) Representative: Reed, Michael Antony
(86) International application number: PCT/US2005/046350
(87) International publication number: WO 2006/066267

(56) References cited:
- WO-A1-99/35146
- BLACKWELL K L ET AL: "Determining molecular phenotypes of metastatic breast cancer that respond to the small molecule inhibitor of ErbB1 and ErbB2, lapatinib (GW572016)" BREAST CANCER RESEARCH AND TREATMENT, vol. 88, no. Suppl. 1, December 2004 (2004-12), page S30, XP008111956 & 27TH ANNUAL CHARLES A COLTMAN SAN ANTONIO BREAST CANCER SYMPOSIUM; SAN ANTONIO, TX, USA; DECEMBER 08 -11, 2004 ISSN: 0167-6806
- LIN N U ET AL: "CNS metastases in breast cancer" JOURNAL OF CLINICAL ONCOLOGY 2004 US, vol. 22, no. 17, 1 September 2004 (2004-09-01), pages 3608-3617, XP008111959 ISSN: 0732-183X
- XIA W ET AL: "Truncated ErbB2 receptor (p95 ErbB2) is regulated by heregulin through heterodimer formation with ErbB3 yet remains sensistive to the dual EGFR/ErbB2 kinase inhibitor Gw572016" ONCOGENE, NATURE PUBLISHING GROUP, GB BASINGSTOKE, HANTS, vol. 23, no. 3, 22 January 2004 (2004-01-22), pages 646-653, XP003000877 ISSN: 0950-9232
- BURRIS H.A. ET AL.: 'Dual Kinase Inhibition in the Treatment of Breast Cancer: Initial Experience with the EGFR/ErbB-2 Inhibitor Lapatinib' THE ONCOLOGIST vol. 9, no. SUPPL. 3, 2004, pages 10 - 15, XP002991589
- KIRSCH ET AL.: 'Targeting HER2 in Brain Metastases from Breast Cancer' CLINICAL CANCER RESEARCH vol. 9, 15 November 2003, pages 5435 - 5436, XP003005591
- LENTZSCH ET AL.: 'Brain Metastases in Breast Cancer: Prognostic Factors and Management' EUROPEAN JOURNAL OF CANCER vol. 35, no. 4, 1999, pages 580 - 585, XP003005592
- SPECTOR ET AL.: 'Study of the Biologic Effects of Lapatinib, a Reversible Inhibitor or ErbB1 and ErbB2 Tyrosine Kinases, on Tumor Growth and Survival Pathways in Patients with Advanced Malignancies' JOURNAL OF CLINICAL ONCOLOGY vol. 23, no. 11, 10 April 2005, pages 2502 - 2512, XP008074364
- RUSNAK ET AL.: 'The Effects of the Novel, Reversible Epidermal Growth Factor Receptor/ErbB-2 Tyrosine Kinase Inhibitor, GW2016, on the Growth of Human Normal and Tumor-Derived Cell Lines in Vitro and in Vivo' MOLECULAR CANCER THERAPEUTICS vol. 1, December 2001, pages 85 - 94, XP002964819
- WOOD ET AL.: 'A Unique Structure for Epidermal Growth Factor Receptor Bound to GW572016 (Lapatinib): Relationships Among Protein Conformation, Inhibitor Off-Rate, and Receptor Activity in Tumor Cells' CANCER RESEARCH vol. 64, 15 September 2004, pages 6652 - 6659, XP003005593
- ZULKOWSKI ET AL.: 'Regression of Brain Metastases from Breast Carcinoma after Chemotherapy with Bendamustine' J CANCER RES CLIN ONCOL vol. 128, 2002, pages 111 - 113, XP003005594
- SLICHENMYER WILLIAM J ET AL: "CI-1033, a pan-erbB tyrosine kinase inhibitor", SEMINARS IN ONCOLOGY, vol. 28, no. 5 Suppl. 16, October 2001 (2001-10), pages 80-85, ISSN: 0093-7754
- STEMMLER HANS-JOACHIM ET AL: "Central nervous system metastases in HER-2-overexpressing metastatic breast cancer: a treatment challenge.", THE ONCOLOGIST JUL 2008, vol. 13, no. 7, July 2008 (2008-07), pages 739-750, ISSN: 1083-7159
- RIXE OLIVIER ET AL: "A randomized, phase II, dose-finding study of the pan-ErbB receptor tyrosine-kinase inhibitor CI-1033 in patients with pretreated metastatic breast cancer", CANCER CHEMOTHERAPY AND PHARMACOLOGY, vol. 64, no. 6, November 2009 (2009-11), pages 1139-1148, ISSN: 0344-5704

## Description

The present invention relates to treating breast cancer that has metastasized to the brain in a mammal by administration of 4-quinazolinamines and pharmaceutical compositions containing the same. In particular, it relates to treating breast cancer brain metastases which overexpress erbB2 by administration of N-{3-chloro-4-[(3-fluorobenzyl) oxy]phenyl}-6-[5-({[2-(methanesulphonyl) ethyl]amino} methyl)-2-furyl]-4-quinazolinamine and salts and solvates thereof.

Trastuzumab-based regimens have improved both systemic control and overall survival in patients with metastatic ErbB2 overexpressing breast cancer. Trastuzumab, however, does not cross the blood-brain barrier and ErbB2-positive breast cancer may have a predilection to metastasize to distant organs, including the the brain. As a result, CNS progression is emerging as a major clinical problem. A recent analysis of 523 metastatic breast cancer patients enrolled in two clinical trials of first-line trastuzumab revealed a 10% incidence of isolated CNS progression, with a higher incidence of CNS disease among patients confirmed to have HER2-overexpressing tumors (Burstein J.C., et al, Breast Cancer Res Treat., 82:S50-S51, 2003, Supp. 1, abstract 226). Furthermore, retrospective analyses have disclosed a 28-43% incidence of CNS metastases among women treated with trastuzumab for stage IV breast cancer across multiple institutions (Bendell J.C., et al, Cancer 97:2972-2977,2003; Weitzen R., et al, Proc. Am. Soc. Clin. Oncol., 21:2002, abstract 1936; Wardley A.M., et al, Proc. Am. Soc. Clin. Oncol., 21:2002, abstract 241; and Heinrich B., Proc. Am. Soc. Clin. Oncol., 22: 2003, abstract 147). In contrast, the incidence of CNS metastases in historical series among unselected patients was only 10-16% (Hagemeister F. B. et al, Cancer 46:162-167, 1980) Importantly, despite CNS treatment such as whole brain radiotherapy (WBRT) or stereotactic radiosurgery (SRS), an increasing proportion of patients are dying of neurologic causes, rather than of progressive systemic disease. In the series by Bendell et al., for example, 71 % of patients were still responding or had achieved stable disease systemically at the time of progression in the CNS, and over half died of neurologic causes (Bendell J. C. et al, Cancer 97:2972-2977, 2003). Effective and well-tolerated therapy for brain metastases from breast cancer remains an unmet medical need.

In general, the initial treatment of brain metastases depends upon their multiplicity, location, size, and upon the status of a patient's systemic disease. Options may include surgical resection, stereotactic radiosurgery (SRS), and whole brain radiotherapy (WBRT). As patients survive longer, CNS progression after WBRT and/or SRS is a significant clinical problem. Currently there is no consensus on optimal treatment once this occurs; possible options are to attempt or re-attempt SRS or to utilize chemotherapy. Few trials have examined the role of chemotherapy in the treatment of brain metastases from breast cancer, and most studies of novel agents for breast cancer have specifically excluded patients with brain metastases. Data is primarily available from case series and case reports. Isolated case reports of activity exist for variety of agents including capecitabine, cisplatin plus etoposide, and bendamustine (Wang M. L. H. et al, Am. J. Clin. Oncol. 24:421-424,2001; Cocconi G. et al, Cancer Invest. 8:327-334, 1990; Franciosi V. et al, Cancer 85:1599-1605, 1999; and Zulkowski K. et al, J. Cancer Res. Clin. Oncol. 128:111-113,2001). Temozolomide is an orally bioavailable alkylating agent that readily crosses the blood-brain barrier. Phase II trials have reported inconsistent activity with temozolomide in this population (Christodoulou C. et al, Ann. Oncol. 12:249-254, 2001). No responses have been described in the literature to paclitaxel, docetaxel, or navelbine, which are among the most widely used chemotherapeutic agents in the treatment of metastatic breast cancer.

The blood-brain barrier (BBB) excludes many therapeutic agents from the CNS. The BBB is formed by the complex tight junctions between the endothelial cells of the brain capillaries and their low endocytic activity (Potschka et al, Journal of Pharm. And Exp. Therapeutics 306(1):124-131, 2003 July). This results in a capillary wall that behaves as a continuous lipid bilayer and prevents the passage of polar and lipid-insoluble substances. Additionally, ATP-dependent multidrug transporters such as P-glycoprotein (Pgp; ABCB1) and multidrug resistance protein MRP2 (ABCC2), which are found in the membranes of brain capillary endothelial cells, are thought to play an important role in BBB function by limiting drug penetration into the brain. It is, therefore, the major obstacle to drugs that may combat diseases affecting the CNS.

Brain tumors may disrupt the function of the BBB locally and nonhomogeneously. As mentioned above, CNS activity with a variety of chemotherapeutic regimens (cytoxan/methotrexate/5-fluorouracil, doxorubicin/cytoxan, capecitabine, cisplatin/etoposide) has been reported, despite the fact that none of these agents cross the intact blood brain barrier at the doses used. Furthermore, in two autopsy studies, clinically relevant concentrations of platins were present within brain tumors, but not adjacent normal brain, supporting the hypothesis that the blood-tumor barrier has very distinct properties than the intact blood brain barrier (Stewart D. J. et al, Am. J. Clin. Oncol. 11:152-158, 1988; and Stewart D. J. et al, Cancer Res. 42:2472-2479). In contrast, the large (Mᵣ 148,000) monoclonal antibody trastuzumab, remains excluded from the CNS in both preclinical xenograft models and in patients with CNS metastases (Grossi P. M. et al, Clin. Cancer Res. 9:5514-5520, 2003; and Pestalozzi B. C. et al, J. Cin. Oncol. 18:2349-2351,2000).

Small molecule tyrosine kinase inhibitors may cross a disrupted blood brain barrier. A 69% partial response rate and 80% systemic disease control rate with first-line gefitinib in Asian, female, non-smokers with NSCLC adenocarcinoma was recently reported (Lee Jun-Soo, International Assoc. for the Study of Lung Cancer, Baltimore, July 2004). Gefitinib (Iressa©) is an inhibitor of the epidermal growth factor receptor (EGFR) and is indicated as monotherapy for the treatment of patients with locally advanced or metastatic non-small cell lung cancer after failure of both platinum-based and docetaxel chemotherapies. This study prospectively included eight subjects with newly diagnosed, untreated non small cell lung cancer (NSCLC) brain metastases. Seven out of eight of these subjects obtained prolonged CNS partial responses with gefitinib. Activity with gefitinib was reported in another prospectively conducted trial in patients with progressive NSCLC brain metastases following chemotherapy +/- WBRT (Ceresoli G. L., Annals Oncol. 15:1042-1047. Additionally, there have been 22 case reports thus far in the literature of CNS responses to gefitinib among subjects with NSCLC treated on a compassionate-use program. Both complete and partial objective responses were noted and associated with improvements in functional status and quality-of-life.

Another potential mechanism of resistance to trastuzumab therapy is a discordance in ErbB2 expression between the primary tumor and sites of metastases e.g. ErbB2 non-overexpressing cells escape systemic therapy with trastuzumab. The transcriptional expression of ErbB2 in brain metastases however, has been shown to routinely exceed that of the primary breast cancer (Steeg P., Third Int'l. Symp. On Translational Res. In Onc., Santa Barbara CA, Oct9-12, 2003; and Steeg P. et al, Eur. J. Cancer 2(8):142, Sept 2004, abstract 468). Alternatively, clones that metastasize to the CNS may be resistant to trastuzumab via mechanisms such as upregulation of the truncated ErbB2 receptor p95 or a PTEN deficiency (Nagata Y. et al, Cancer Cell 6(2):117-127, 2004; and Christianson T. A. et al, Cancer Research 58(22):5123-5129, 1998). As indicated above, brain metastases typically develop while systemic disease is well controlled with trastuzumab. Hence, the most reasonable assumption is the large monoclonal antibody trastuzumab cannot access the CNS regardless of the blood-brain-barrier status.

In the editorial article by Kirsch and Hochberg.: 'Targeting HER2 in Brain Metastases from Breast Cancer', Clinical Cancer Research, Vol. 9, 15 November 2003, pages 5435-5436 it is disclosed that metastatic breast cancer is routinely treated with trastuzumab. The article, however, further indicates that the breast cancer frequently progresses in the CNS after treatment with trastuzumab, probably resulting from the poor penetration of trastuzumab into the brain. The article describes the use of cerebral microinfusion of trastuzumab directly into the brain of rats in order to overcome the limitations caused by the inability of trastuzumab to cross the blood-brain barrier. The article also proposes that small molecules that cross the blood-brain barrier and inhibit HER2 tyrosine kinase activity may be attractive drugs to treat brain metastases from HER2-overexpressing breast cancer. The authors suggest that the two small molecule HER2 inhibitors CI-1033 and GW572016 might be able to cross the blood-brain barrier.

The present inventor has now identified novel treatment methods for breast cancer that has metastasized to the brain. Such method includes administration of N-{3-chloro-4-[(3-fluorobenzyl) oxy]phenyl}-6-[5-({[2-(methanesulphonyl) ethyl]amino}methyl)-2-furyl]-4-quinazolinamine (GW572016) as well as salts and/or solvates thereof to a mammal in need thereof.

### SUMMARY OF THE INVENTION

In a first aspect of the present invention, there is provided the use of therapeutically effective amounts of a compound of formula (I) 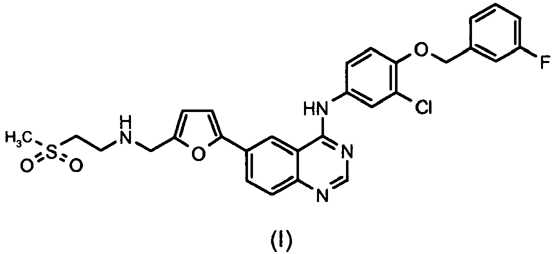 or a pharmaceutically acceptable salt thereof in the preparation of a medicament for the treatment in a mammal of breast cancer metastatic sites in the brain.

In a second aspect of the present invention there is provided a compound of formula (I) 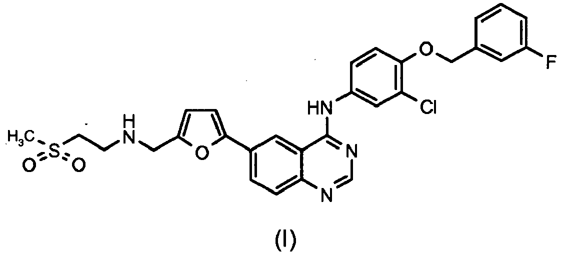 or a pharmaceutically acceptable salt thereof for use in the treatment in a mammal of breast cancer metastatic sites in the brain.

In an embodiment the compound of formula (I) or a pharmaceutically acceptable salt thereof is lapatinib ditosylate, a compound of formula (I')

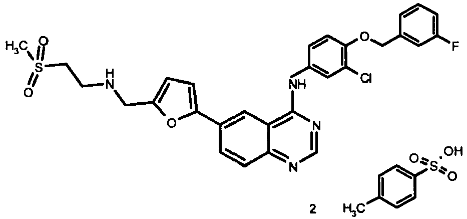

In an embodiment the compound of formula (I) or a pharmaceutically acceptable salt thereof is lapatinib ditosylate monohydrate, a compound of formula (I")

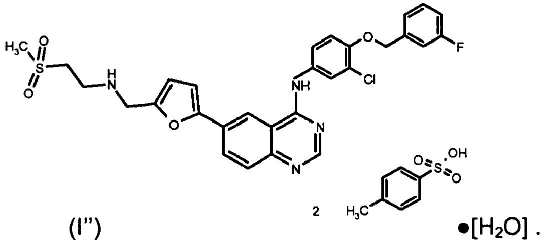

### DETAILED DESCRIPTION OF THE INVENTION

As used herein the term "neoplasm" refers to an abnormal growth of cells or tissue and is understood to include benign, i.e., non-cancerous growths, and malignant, i.e., cancerous growths including primary or metastatic cancerous growths. The term "neoplastic" means of or related to a neoplasm.

"EGFR", also known as "erbB-1", and "erbB-2" are protein tyrosine kinase transmembrane growth factor receptors of the erbB family. Protein tyrosine kinases catalyse the phosphorylation of specific tyrosyl residues in various proteins involved in the regulation of cell growth and differentiation (A.F. Wilks, Progress in Growth Factor Research, 1990, 2, 97-111; S.A. Courtneidge, Dev. Supp.I, 1993, 57-64; J.A. Cooper, Semin. Cell Biol., 1994, 5(6), 377-387; R.F. Paulson, Semin. Immunol., 1995, 7(4), 267-277; A.C. Chan, Curr. Opin. Immunol., 1996, 8(3), 394-401). The ErbB family of type I receptor tyrosine kinases includes ErbB1 (also known as the epidermal growth factor receptor (EGFR or HER1)), ErbB2 (also known as Her2), ErbB3, and ErbB4. These receptor tyrosine kinases are widely expressed in epithelial, mesenchymal, and neuronal tissues where they play a role in regulating cell proliferation, survival, and differentiation (Sibilia and Wagner, Science, 269: 234 (1995); Threadgill et al., Science, 269: 230 (1995)). Increased expression of wild-type ErbB2 or EGFR, or expression of constitutively activated receptor mutants, transforms cells *in vitro* (Di Fiore *et al*., 1987; DiMarco et al, Oncogene, 4: 831 (1989); Hudziak et al., Proc. Natl. Acad. Sci. USA., 84:7159 (1987); Qian et al., Oncogene, 10:211 (1995)). Increased expression of ErbB2 or EGFR has been correlated with a poorer clinical outcome in some breast cancers and a variety of other malignancies (Slamon et al., Science, 235: 177 (1987); Slamon et al., Science, 244:707 (1989); Bacus et al, Am. J. Clin. Path., 102:S13 (1994)).

As used herein, a cell "overexpressing" ErbB2 refers to a cell having a significantly increased number of functional ErbB2 receptors, compared to the average number of receptors that would be found on a cell of that same type. Overexpression of ErbB2 has been documented in various cancer types, including breast (Verbeek et al., FEBS Letters 425:145 (1998); colon (Gross et al., Cancer Research 51:1451 (1991)); lung (Damstrup et al., Cancer Research 52:3089 (1992), renal cell (Stumm et al, Int. J. Cancer 69:17 (1996), Sargent et al., J. Urology 142: 1364 (1989)) and bladder (Chow et al., Clin. Cancer Res. 7:1957 (2001); Bue et al., Int. J. Cancer, 76:189 (1998); Turkeri et al., Urology 51: 645 (1998)). Overexpression of ErbB2 may be assessed by any suitable method as is known in the art, including but not limited to imaging, gene amplification, number of cell surface receptors present, protein expression, and mRNA expression. See e.g., Piffanelli et al., Breast Cancer Res. Treatment 37:267 (1996).

As used herein, the term "effective amount" means that amount of a drug or pharmaceutical agent that will elicit the biological or medical response of a tissue, system, animal or human that is being sought, for instance, by a researcher or clinician. Furthermore, the term "therapeutically effective amount" means any amount which, as compared to a corresponding subject who has not received such amount, results in improved treatment, healing, or amelioration of a disease, disorder, or side effect, or a decrease in the rate of advancement of a disease or disorder. The term also includes within its scope amounts effective to enhance normal physiological function.

As used herein, the term "solvate" refers to a complex of variable stoichiometry formed by a solute (in this invention, compounds of formula (I) or a salt thereof) and a solvent. Such solvents for the purpose of the invention may not interfere with the biological activity of the solute. Examples of suitable solvents include, water, methanol, ethanol and acetic acid. Preferably the solvent used is a pharmaceutically acceptable solvent. Examples of suitable pharmaceutically acceptable solvents include, water, ethanol and acetic acid. Most preferably the solvent used is water.

The use in cancer treatment disclosed herein includes use of a compound of formula (I): 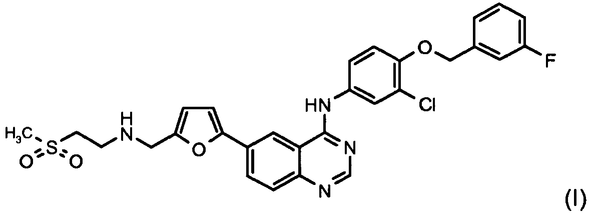 or a pharmaceutically acceptable salt thereof.

In another embodiment, the compound is a compound of formula (I') which is the ditosylate salt of the compound of formula (I) and anhydrate or hydrate forms thereof. The ditosylate salt of the compound of formula (I) has the chemical name N-{3-chloro-4-[(3-fluorobenzyl) oxy]phenyl}6-[5-({[2-(methanesulphonyl) ethyl]amino}methyl)-2-furyl]-4-quinazolinamine (GW572016) ditosylate and is also known as lapatinib ditosylate.

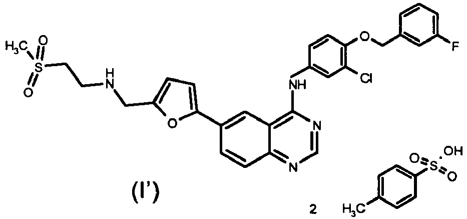

In one embodiment, the compound is the anhydrous ditosylate salt of the compound of formula (I'). In another embodiment, the compound is a compound of formula (I") which is the monohydrate ditosylate salt of the compound of formula (I').

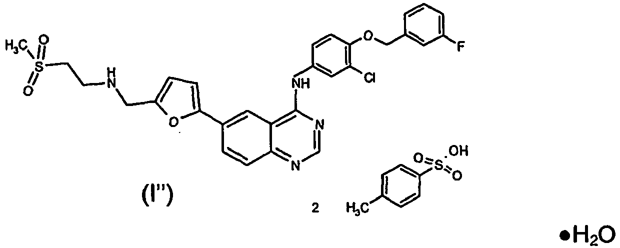

The free base, HCl salts, and ditosylate salts of the compound of Formula (I) may be prepared according to the procedures of International Patent Application No. PCT/EP99/00048, filed January 8, 1999, and published as WO 99/35146 on July 15, 1999, referred to above and International Patent Application No. PCT/US01/20706, filed June 28, 2001 and published as WO 02/02552 on January 10, 2002 and according to the appropriate Examples recited below. One such procedure for preparing the ditosylate salt of the compound of formula (I) is presented following in Scheme 1.

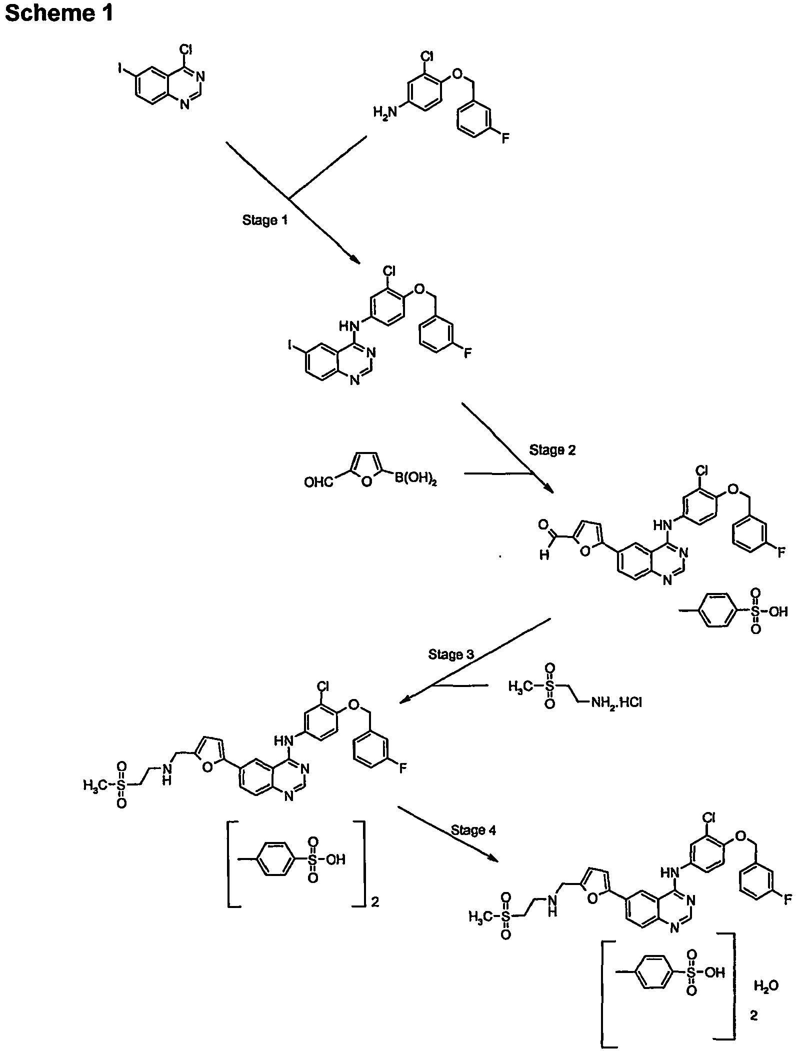

In scheme 1, the preparation of the ditosylate salt of the compound of formula (III) proceeds in four stages: Stage 1: Reaction of the indicated bicyclic compound and amine to give the indicated iodoquinazoline derivative; Stage 2: preparation of the corresponding aldehyde salt; Stage 3: preparation of the quinazoline ditosylate salt; and Stage 4: monohydrate ditosylate salt preparation.

The salts of the present invention are pharmaceutically acceptable salts. Salts encompassed within the term "pharmaceutically acceptable salts" refer to non-toxic salts of the compounds of this invention. Salts of the compounds of the present invention may comprise acid addition salts derived from a nitrogen on a substituent in a compound of the present invention. Representative salts include the following salts: acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, laurate, malate, maleate, mandelate, mesylate, methylbromide, methylnitrate, methylsulfate, monopotassium maleate, mucate, napsylate, nitrate, N-methylglucamine, oxalate, pamoate (embonate), palmitate, pantothenate, phosphate/diphosphate, polygalacturonate, potassium, salicylate, sodium, stearate, subacetate, succinate, tannate, tartrate, teoclate, tosylate, triethiodide, trimethylammonium and valerate. Other salts, which are not pharmaceutically acceptable, may be useful in the preparation of compounds of this invention.

While it is possible that, for use in the treatment of cancer according to the present invention therapeutically effective amounts of a compound of formula (I) as well as pharmaceutically acceptable salts thereof, may be administered as the raw chemical, it is possible to present the active ingredient as a pharmaceutical composition. Such pharmaceutical compositions may be administered in the treatment of cancer according to the present invention. The pharmaceutical compositions include therapeutically effective amounts of a compound of formula (I) or a pharmaceutically acceptable salt thereof, and one or more pharmaceutically acceptable carriers, diluents, or excipients. The carrier(s), diluent(s) or excipient(s) must be acceptable in the sense of being compatible with the other ingredients of the formulation and not deleterious to the recipient thereof.

Pharmaceutical formulations may be presented in unit dose forms containing a predetermined amount of active ingredient per unit dose. Such a unit may contain, for example, 0.5mg to 1 g, preferably 1mg to 700mg, more preferably 5mg to 100mg of a compound of formula (I), depending on the condition being treated, the route of administration and the age, weight and condition of the patient, or pharmaceutical formulations may be presented in unit dose forms containing a predetermined amount of active ingredient per unit dose. Preferred unit dosage formulations are those containing a daily dose or sub-dose, as herein above recited, or an appropriate fraction thereof, of an active ingredient. Furthermore, such pharmaceutical formulations may be prepared by any of the methods well known in the pharmacy art.

The compound of formula (I) may be administered by any appropriate route. Suitable routes include oral, rectal, nasal, topical (including buccal and sublingual), vaginal, and parenteral (including subcutaneous, intramuscular, intraveneous, intradermal, intrathecal, and epidural). It will be appreciated that the preferred route may vary with, for example, the condition of the recipient of the combination.

The use and compound for the treatment of cancer according to the present invention may also be employed with other therapeutic cancer treatment agents. In particular, in anti-neoplastic therapy, combination therapy with other chemotherapeutic, hormonal, antibody agents as well as surgical and/or radiation treatments other than those mentioned above are envisaged. Anti-neoplastic therapies are described for instance in International Application No. PCT US 02/01130, filed January 14, 2002, which application is incorporated by reference to the extent that it discloses anti-neoplastic therapies. Combination therapies according to the present invention thus include the administration of at least one compound of formula (I) as well as optional use of other therapeutic agents including other anti-neoplastic agents. Such combination of agents may be administered together or separately and, when administered separately this may occur simultaneously or sequentially in any order, both close and remote in time. The amounts of the compound of formula (I) and the other pharmaceutically active agent(s) and the relative timings of administration will be selected in order to achieve the desired combined therapeutic effect.

Pharmaceutical formulations adapted for oral administration may be presented as discrete units such as capsules or tablets; powders or granules; solutions or suspensions in aqueous or non-aqueous liquids; edible foams or whips; or oil-in-water liquid emulsions or water-in-oil liquid emulsions.

For instance, for oral administration in the form of a tablet or capsule, the active drug component can be combined with an oral, non-toxic pharmaceutically acceptable inert carrier such as ethanol, glycerol, water. Powders are prepared by comminuting the compound to a suitable fine size and mixing with a similarly comminuted pharmaceutical carrier such as an edible carbohydrate, as, for example, starch or mannitol. Flavoring, preservative, dispersing and coloring agent can also be present.

Capsules are made by preparing a powder mixture as described above, and filling formed gelatin sheaths. Glidants and lubricants such as colloidal silica, talc, magnesium stearate, calcium stearate or solid polyethylene glycol can be added to the powder mixture before the filling operation. A disintegrating or solubilizing agent such as agar-agar, calcium carbonate or sodium carbonate can also be added to improve the availability of the medicament when the capsule is ingested.

Moreover, when desired or necessary, suitable binders, lubricants, disintegrating agents and coloring agents can also be incorporated into the mixture. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, waxes and the like. Lubricants used in these dosage forms include sodium oleate, sodium stearate, magnesium stearate, sodium benzoate, sodium acetate, sodium chloride and the like. Disintegrators include, starch, methyl cellulose, agar, bentonite, xanthan gum. Tablets are formulated, for example, by preparing a powder mixture, granulating or slugging, adding a lubricant and disintegrant and pressing into tablets. A powder mixture is prepared by mixing the compound, suitably comminuted, with a diluent or base as described above, and optionally, with a binder such as carboxymethylcellulose, an aliginate, gelatin, or polyvinyl pyrrolidone, a solution retardant such as paraffin, a resorption accelerator such as a quaternary salt and/or an absorption agent such as bentonite, kaolin or dicalcium phosphate. The powder mixture can be granulated by wetting with a binder such as syrup, starch paste, acadia mucilage or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc or mineral oil. The lubricated mixture is then compressed into tablets. The compounds of the present invention can also be combined with free flowing inert carrier and compressed into tablets directly without going through the granulating or slugging steps. A clear or opaque protective coating consisting of a sealing coat of shellac, a coating of sugar or polymeric material and a polish coating of wax can be provided. Dyestuffs can be added to these coatings to distinguish different unit dosages.

Oral fluids such as solution, syrups and elixirs can be prepared in dosage unit form so that a given quantity contains a predetermined amount of the compound. Syrups can be prepared by dissolving the compound in a suitably flavored aqueous solution, while elixirs are prepared through the use of a non-toxic alcoholic vehicle. Suspensions can be formulated by dispersing the compound in a non-toxic vehicle. Solubilizers and emulsifiers such as ethoxylated isostearyl alcohols and polyoxy ethylene sorbitol ethers, preservatives, flavor additive such as peppermint oil or natural sweeteners or saccharin or other artificial sweeteners can also be added.

Where appropriate, dosage unit formulations for oral administration can be microencapsulated. The formulation can also be prepared to prolong or sustain the release as for example by coating or embedding particulate material in polymers, wax or the like.

The agents for use according to the present invention can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

Agents for use according to the present invention may also be delivered by the use of monoclonal antibodies as individual carriers to which the compound molecules are coupled. The compounds may also be coupled with soluble polymers as targetable drug carriers. Such polymers can include polyvinylpyrrolidone, pyran copolymer, polyhydroxypropylmethacrylamide-phenol, polyhydroxyethylaspartamidephenol, or polyethyleneoxidepolylysine substituted with palmitoyl residues. Furthermore, the compounds may be coupled to a class of biodegradable polymers useful in achieving controlled release of a drug, for example, polylactic acid, polepsilon caprolactone, polyhydroxy butyric acid, polyorthoesters, polyacetals, polydihydropyrans, polycyanoacrylates and cross-linked or amphipathic block copolymers of hydrogels.

Pharmaceutical formulations adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3(6), 318 (1986).

Pharmaceutical formulations adapted for topical administration may be formulated as ointments, creams, suspensions, lotions, powders, solutions, pastes, gels, sprays, aerosols or oils.

For treatments of the eye or other external tissues, for example mouth and skin, the formulations are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base.

Pharmaceutical formulations adapted for topical administrations to the eye include eye drops wherein the active ingredient is dissolved or suspended in a suitable carrier, especially an aqueous solvent.

Pharmaceutical formulations adapted for topical administration in the mouth include lozenges, pastilles and mouth washes.

Pharmaceutical formulations adapted for rectal administration may be presented as suppositories or as enemas.

Pharmaceutical formulations adapted for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable formulations wherein the carrier is a liquid, for administration as a nasal spray or as nasal drops, include aqueous or oil solutions of the active ingredient.

Pharmaceutical formulations adapted for administration by inhalation include fine particle dusts or mists that may be generated by means of various types of metered dose pressurised aerosols, nebulizers or insufflators.

Pharmaceutical formulations adapted for vaginal administration may be presented as pessaries, tampons, creams, gels, pastes, foams or spray formulations.

Pharmaceutical formulations adapted for parenteral administration include aqueous and non-aqueous sterile injection solutions which may contain antioxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets.

It should be understood that in addition to the ingredients particularly mentioned above, the formulations may include other agents conventional in the art having regard to the type of formulation in question, for example those suitable for oral administration may include flavoring agents.

As indicated, therapeutically effective amounts of a specific compound of formula (I) is administered to a mammal. Typically, the therapeutically effective amount of one of the administered agents of the present invention will depend upon a number of factors including, for example, the age and weight of the mammal, the precise condition requiring treatment, the severity of the condition, the nature of the formulation, and the route of administration. Ultimately, the therapeutically effective amount will be at the discretion of the attendant physician or veterinarian.

Typically, the compound of formula (I) will be given in the range of 0.1 to 100 mg/kg body weight of recipient (mammal) per day and more usually in the range of 1 to 10 mg/kg body weight per day.

As recited above the present invention is directed to compounds for use in treatment method for breast cancer that has metastasized to the brain. Such method includes administration of N-{3-chloro-4-[(3-fluorobenzyl) oxy]phenyl}-6-[5-({[2-(methanesulphonyl) ethyl]amino}methyl)-2-furyl]-4-quinazolinamine (GW572016) as well as pharmaceutically acceptable salts thereof to a mammal in need thereof.

In one embodiment the brain metastatic sites overexpress erbB-2.

In another embodiment, the mammal has previously been treated with trastuzumab.

In one embodiment, the compound of formula (I) is a compound of formula (I'). In another embodiment, the compound of formula (I) is the compound of formula (I").

It is also disclosed, but not part of the invention that the compounds of formulae (I), (I'), and (I"), if utilized to treat earlier stages of breast cancer alone or in combination with other anti-neoplastics, may prevent or delay the development of brain metastases.

In the foregoing uses in cancer treatment according to the present invention the compounds of formulae (I), (I'), and (I") are as described above.

It is contemplated that a compound of formulae (I), (I'), or (I") and at least one additional cancer treatment therapy may be employed in the disclosed cancer treatment methods. The compounds of formulae (I), (I'), and (I") may be utilized in combination concomitantly or sequentially in any therapeutically appropriate combination with such other anti-cancer therapies. In one embodiment, the additional anti-cancer therapy is a radiation therapy, including stereotactic radiosurgery (SRS) and whole brain radiotherapy (WBRT). In one embodiment, the other anti-cancer therapy is at least one additional chemotherapeutic therapy including administration of at least one anti-neoplastic agent. The administration in combination of a compound of formula (I) or a pharmaceutically acceptable salt thereof with other anti-neoplastic agents may be in combination in accordance with the invention by administration concomitantly in (1) a unitary pharmaceutical composition including both compounds, or (2) separate pharmaceutical compositions each including one of the compounds. Alternatively, the combination may be administered separately in a sequential manner wherein one anti-neoplastic agent is administered first and the other second or vice versa. Such sequential administration may be close in time or remote in time.

Anti-neoplastic agents may induce anti-neoplastic effects in a cell-cycle specific manner, i.e., are phase specific and act at a specific phase of the cell cycle, or bind DNA and act in a non cell-cycle specific manner, i.e., are non-cell cycle specific and operate by other mechanisms.

Anti-neoplastic agents useful in combination with the compounds and salts, solvates or physiologically functional derivatives thereof of formula I include the following:
(1) cell cycle specific anti-neoplastic agents including, diterpenoids such as paclitaxel and its analog docetaxel; vinca alkaloids such as vinblastine, vincristine, vindesine, and vinorelbine; epipodophyllotoxins such as etoposide and teniposide; gemcitabine; capecitabine, fluoropyrimidines such as 5-fluorouracil and fluorodeoxyuridine ; antimetabolites such as allopurinol, fludurabine, methotrexate, cladrabine, cytarabine, mercaptopurine and thioguanine; and camptothecins such as 9-amino camptothecin, irinotecan, topotecan, CPT-11 and the various optical forms of 7-(4-methylpiperazino-methylene)-10,11-ethylenedioxy-20-camptothecin;
(2) cytotoxic chemotherapeutic agents including, alkylating agents such as melphalan, chlorambucil, cyclophosphamide, mechlorethamine, hexamethylmelamine, busulfan, carmustine, lomustine, and dacarbazine; anti-tumour antibiotics such as doxorubicin, daunomycin, epirubicin, idarubicin, mitomycin-C, dacttinomycin and mithramycin; and platinum coordination complexes such as cisplatin, carboplatin, and oxaliplatin; and
(3) other chemotherapeutic agents including, anti-estrogens such as tamoxifen, toremifene, raloxifene, droloxifene and iodoxyfene; progestrogens such as megestrol acetate; aromatase inhibitors such as anastrozole, letrazole, vorazole, and exemestane; antiandrogens such as flutamide, nilutamide, bicalutamide, and cyproterone acetate; LHRH agonists and antagagonists such as goserelin acetate and luprolide, testosterone 5α-dihydroreductase inhibitors such as finasteride; metalloproteinase inhibitors such as marimastat; antiprogestogens; urokinase plasminogen activator receptor function inhibitors; Bcl-2 inhibitors, growth factor function inhibitors such as inhibitors of the functions of hepatocyte growth factor; erb-B2, erb-B4, epidermal growth factor receptor (EGFR) including gefitinib and elotinib, platelet derived growth factor receptor (PDGFR), insulin growth factor receptor (IGFR), vascular endothelial growth factor receptor (VEGFR, and TIE-2 (other than those VEGFR and TIE-2 inhibitors described in the present invention); and other kinase inhibitors such as inhibitors of CDK2, CDK4, Akt, c-raf, b-raf, Aurora and Bcr-Abl inhibitors such as imatinib mesylate (Gleevec®).

Accordingly, in one embodiment, the compound for use in methods of the present application may be administered in combination with at least one additional anti-neoplastic compound. In one embodiment, the at least one additional anti-neoplastic is trastuzumab.

In another aspect of the present invention it is contemplated that the compound for use in methods of the present invention may be administered in combination with a compound of formulae (I), (I'), or (I") and an inhibitor of transport proteins such as p-glycoprotein (P-gp) and breast cancer resistant protein (BCRP). A suitable example includes elacridar which is described in U.S. Patents 5,604,237, 6,469,022, 6,803,373, and International Patent Application PCT/NL00/00331 filed May 17, 2000 and published as WO 00/69390 on November 23, 2000.

The following examples are intended for illustration only.

### EXAMPLES

As used herein the symbols and conventions used in these processes, schemes and examples are consistent with those used in the contemporary scientific literature, for example, the *Journal of the American Chemical Society* or the *Journal of Biological Chemistry.* Standard single-letter or three-letter abbreviations are generally used to designate amino acid residues, which are assumed to be in the L-configuration unless otherwise noted. Unless otherwise noted, all starting materials were obtained from commercial suppliers and used without further purification. Specifically, the following abbreviations may be used in the examples and throughout the specification:

| | |
|---|---|
| g (grams); | mg (milligrams); |
| L (liters); | mL (milliliters); |
| µL (microliters); | psi (pounds per square inch); |
| M (molar); | mM (millimolar); |
| N (Normal) | Kg (kilogram) |
| i. v. (intravenous); | Hz (Hertz); |
| MHz (megahertz); | mol (moles); |
| mmol (millimoles); | RT (room temperature); |
| min (minutes); | h (hours); |
| mp (melting point); | TLC (thin layer chromatography); |
| Tᵣ (retention time); | RP (reverse phase); |
| DCM (dichloromethane); | DCE (dichloroethane); |
| DMF (*N*,*N*-dimethylformamide); | HOAc (acetic acid); |
| TMSE (2-(trimethylsilyl)ethyl); | TMS (trimethylsilyl); |
| TIPS (triisopropylsilyl); | TBS (*t*-butyldimethylsilyl); |
| HPLC (high pressure liquid chromatography); | |
| THF (tetrahydrofuran); | DMSO (dimethylsulfoxide); |
| EtOAc (ethyl acetate); | DME (1,2-dimethoxyethane); |
| EDTA | ethylenediaminetetraacetic acid |
| FBS | fetal bovine serum |
| IMDM | Iscove's Modified Dulbecco's medium |
| PBS | phosphate buffered saline |
| RPMI | Roswell Park Memorial Institute |
| RIPA buffer | * |
| RT | room temperature |

| | |
|---|---|
| *150 mM NaCl, 50 mM Tris-HCl, pH 7.5, 0.25% (w/v) -deoxycholate, 1% NP-40, 5 mM sodium orthovanadate, 2 mM sodium fluoride, and a protease inhibitor cocktail. | |

Unless otherwise indicated, all temperatures are expressed in °C (degrees Centigrade). All reactions conducted under an inert atmosphere at room temperature unless otherwise noted.

**GW572016F** is **lapatanib** whose chemical name is N-{3-Chloro-4-[(3-fluorobenzyl)oxy]phenyl)-6-[5-({[2-(methane sulphonyl) ethyl]amino}methyl)-2-furyl]-4-quinazolinamine ditosylate monhydrate.

### Example 1

### Preparation of GW572016F

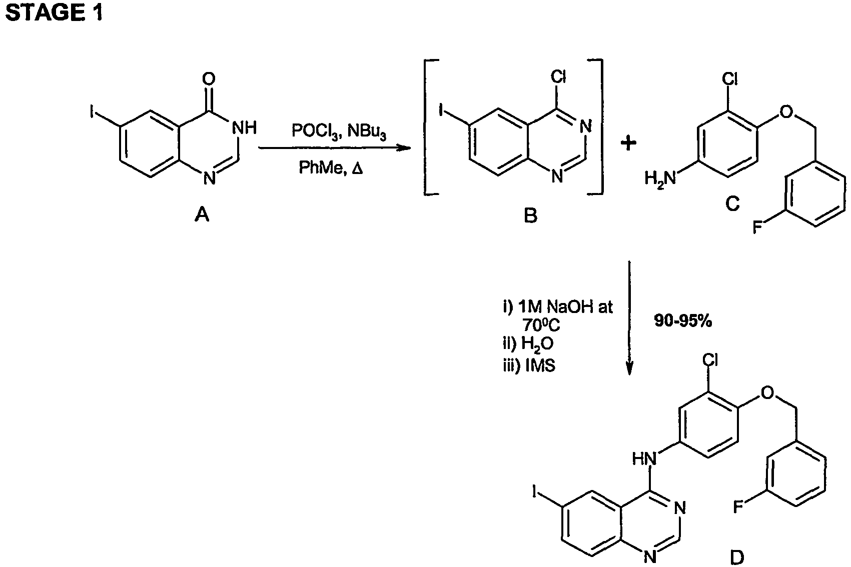

A stirred suspension of 3H-6-iodoquinazolin-4-one (compound A) in toluene (5 vols) was treated with tri-n-butylamine (1.2 eq.) at 20 to 25°C, then heated to 90°C. Phosphorous oxychloride (1.1eq) was added, the reaction mixture was then heated to reflux. The reaction mixture was cooled to 50°C and toluene (5vols) added. Compound C (1.03 eq.) was added as a solid, the slurry was warmed back to 90°C and stirred for 1 hour. The slurry was transferred to a second vessel; the first vessel was rinsed with toluene (2vol) and combined with the reaction mixture. The reaction mixture was cooled to 70°C and 1.0 M aqueous sodium hydroxide solution (16 vols) added dropwise over 1 hour to the stirred slurry maintaining the contents temperature between 68-72°C. The mixture was stirred at 65-70°C for 1 hour and then cooled to 20°C over 1 hour. The suspension was stirred at 20°C for 2 hours, the product collected by filtration, and washed successively with water (3 x 5 vols) and ethanol (IMS, 2 x 5 vols), then dried in vacuo at 50-60°C.

Volumes are quoted with respect of the quantity of Compound A used. **Percent yield range observed:** 90 to 95% as white or yellow crystals.

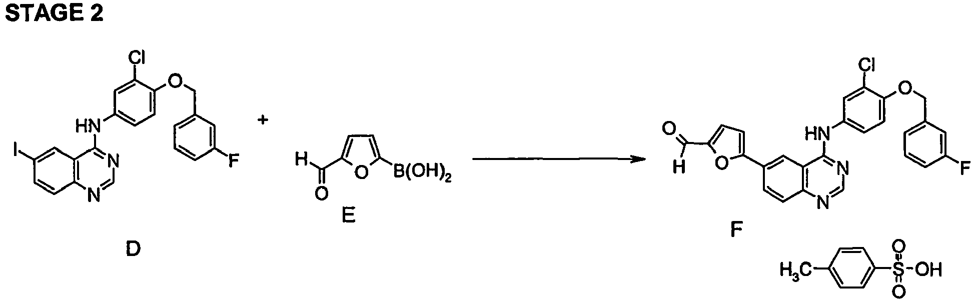

A mixture of N-{3-chloro-4-[(3-fluorobenzyl)oxy]phenyl}-6-iodo-4-quinazolinamine - compound D (1wt), boronic acid - compound E (0.37wt , 1.35equiv), and 10% palladium on charcoal (0.028wt ,50% water wet) was slurried in IMS (15vol). The resultant suspension was stirred for 5 minutes, treated with di-isopropylethylamine (0.39vol, 1.15equiv) and then heated to ca 70°C for ca 3 hours when the reaction was complete (determined by HPLC analysis). The mixture was diluted with tetrahydrofuran (THF, 15vol) and then hot-filtered to remove the catalyst. The vessel was rinsed with IMS (2vol).

A solution of p-toluenesulfonic acid monohydrate (1.5wt, 4 equiv) in water (1.5vol) was added over 5-10 minutes to the filtered solution maintained at 65°C. After crystallisation the suspension was stirred at 60°-65°C for 1 hour, cooled to ca 25°C over 1 hour and stirred at this temperature for a further 2 hours. The solid was collected by filtration, washed with IMS (3vol) then dried *in vacuo* at ca 50°C to give the desired compound F as a yellow-orange crystalline solid (isolated as the ethanol solvate containing approximately 5%w/w EtOH).

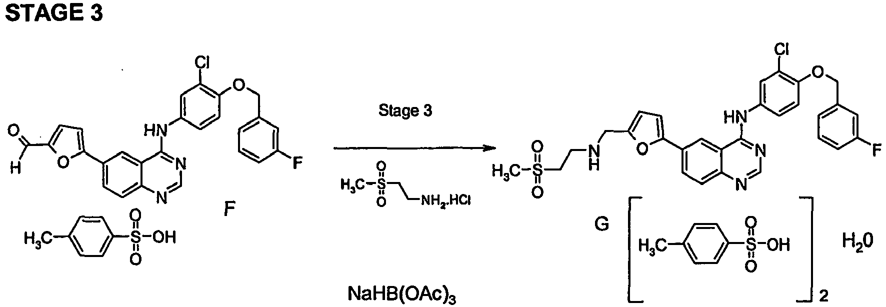

Compound F 1 wt) and 2-(methylsulfonyl)ethylamine hydrochloride (0.4 wt, *1.62* equiv.) were suspended in THF (10 vols). Sequentially, acetic acid (0.354 vol., 4 equiv.) and di-isopropylethylamine (DIPEA, 1.08 vol., *4.01* equiv.) were added. The resulting solution was stirred at 30°-35°C for ca 1 hour then cooled to ca 22°C. Sodium tri-acetoxyborohydride (0.66 wt, *2.01* equiv.) was then added as a continual charge over approximately 15 minutes (some effervescence is seen at this point). The resulting mixture was stirred at ca 22°C for ca 2 hours then sampled for HPLC analysis. The reaction was quenched by addition of aqueous sodium hydroxide (25% w/w, 3 vols.) followed by water (2 vols.) and stirred for ca 30 minutes (some effervescence was seen at the start of the caustic addition).

The aqueous phase was then separated, extracted with THF (2 vols) and the combined THF extracts were then washed twice with 25% w/v aqueous ammonium chloride solution (2 x 5 vols)². A solution of *p*-toluenesulfonic acid monohydrate (p-TSA, 0.74 wt, 2.5 equiv.) in water (1 vol)¹ was prepared, warmed to ca 60°C, and GW572016F (Compound G) (0.002 wt) seeds were added.

The THF solution of the free base of GW572016 was added to the p-TSA solution over at least 30 minutes, while maintaining the batch temperature at 60±3°C. The resulting suspension was stirred at ca 60°C for 1-2 hours, cooled to 20-25°C over an hour and aged at this temperature for ca 1 hr. The solid was collected by filtration, washed with 95:5 THF:Water (3 x 2 vols) and dried *in vacuo* at ca 35°C to give GW572016F - compound G as a bright yellow crystalline solid. Expected yield 80% theory, 117% w/w.
¹ Minimum reaction volume ca 1 vol.
² Maximum reaction volume ca 17 vol.
# Corrected for assay.

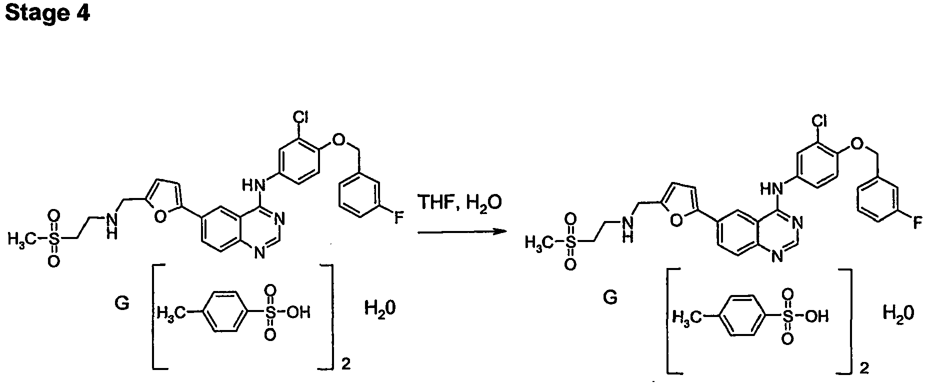

A suspension of the ditosylate monohydrate salt of N-{3-Chloro-4-[(3-fluorobenzyl)oxy]phenyl}-6-[5-({[2-(methane sulphonyl) ethyl]amino}methyl)-2-furyl]-4-quinazolinamine - compound G (1 wt), in tetrahydrofuran (THF, 14 vol) and water (6 vol) was heated to ca 55°-60°C for 30 minutes to give a solution which was clarified by filtration and the lines washed into the crystallisation vessel with THF/Water (7:3 ratio, 2 vol). The resultant solution was heated to reflux and tetrahydrofuran (9 vol, 95% w/w azeotrope with water) was distilled off at atmospheric pressure.

The solution was seeded with N-{3-Chloro-4-[(3-fluorobenzyl)oxy]phenyl}-6-[5-({[2-(methane sulphonyl) ethyl]amino}methyl)-2-furyl]-4-quinazolinamine ditosylate monohydrate (0.002 wt). Once the crystallisation was established water (6 vol) was added while maintaining the reaction temperature above 55°C. The mixture was cooled to 5°-15°C over ca 2 hours. The solid was collected by filtration, washed with tetrahydrofuran/water (3:7 ratio, 2 vol) then tetrahydrofuran/water (19:1 ratio, 2 vol) and dried *in vacuo* at 45°C to give N-{3-Chloro-4-[(3-fluorobenzyl)oxy]phenyl}-6-[5-({[2-(methane sulphonyl) ethyl] amino}methyl)-2-furyl]-4-quinazolinamine ditosylate monohydrate as a bright yellow crystalline solid.

### Example 2

### Clinical study of orally administered lapatinib as single-agent, second-line treatment of patients with brain metastases from erbB-2 overexpressing, metastatic (stage IV) breast cancer

In an on-going clinical study patients with brain metastases from erbB-2 overexpressing, metastatic breast cancer, who had previously been treated with trastuzumab, received and continue to receive 750 mg lapatinib twice daily subject to toxicity, disease progression or withdrawal. Safety and efficacy assessments (independent review) were to be carried out at 2 and 4-week intervals respectively. PET scans were to be run at baseline, 1 week and 8 weeks and MRI at baseline, 8 weeks, and 16 weeks.

The initial week 1 PET scan results on one patient showed promising activity with a dramatic change in one lesion while other brain lesions showed less change or no change at all. The patient had disease which had progressed through Xeloda® (capecitabine), Navelbine® (vinorelbine)/Herceptin® (trastuzumab), and single agent Herceptin® (trastuzumab) treatments. She entered the study with progression in the liver and the brain. The initial one week PET scans showed promising activity after treatment with lapatinib.

## Claims

1. The use of therapeutically effective amounts of a compound of formula (I) 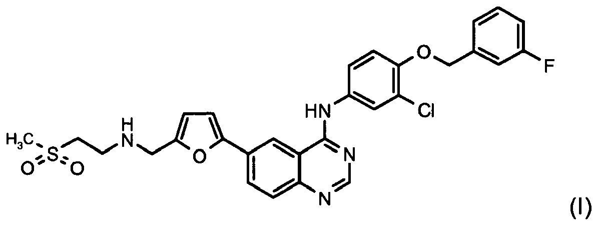 or a pharmaceutically acceptable salt thereof in the preparation of a medicament for the treatment in a mammal of breast cancer metastatic sites in the brain.

2. The use of claim 1 wherein the breast cancer brain metastatic sites overexpress erbB-2.

3. The use as claimed in claim 1 or claim 2 wherein the compound of formula (I) or a pharmaceutically acceptable salt thereof is lapatinib ditosylate.

4. The use as claimed in claim 1 or claim 2 wherein the compound of formula (I) or a pharmaceutically acceptable salt thereof is lapatinib ditosylate monohydrate.

5. A compound of formula (I) 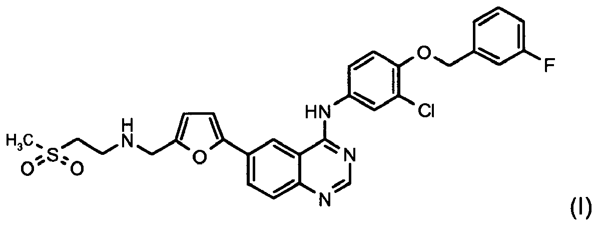 or a pharmaceutically acceptable salt thereof for use in the treatment in a mammal of breast cancer metastatic sites in the brain.

6. The compound of claim 5 for use as claimed in claim 5 wherein the breast cancer brain metastatic sites overexpress erbB-2.

7. The compound of claim 5 for use as claimed in claim 5 or claim 6 wherein the compound of formula (I) or a pharmaceutically acceptable salt thereof is lapatinib ditosylate.

8. The compound of claim 5 for use as claimed in claim 5 or claim 6 wherein the compound of formula (I) or a pharmaceutically acceptable salt thereof is lapatinib ditosylate monohydrate.

## Patentansprüche

1. Die Verwendung therapeutisch wirksamer Mengen einer Verbindung der Formel (I) 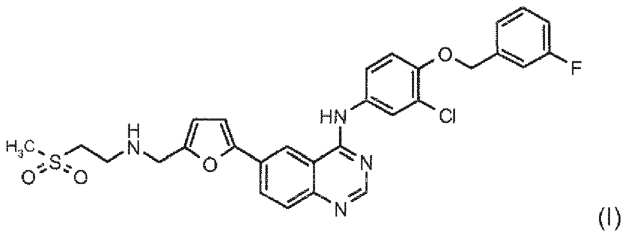 oder eines pharmazeutisch verträglichen Salzes davon zur Herstellung eines Medikaments zur Behandlung von metastatischen Stellen von Brustkrebs im Gehirn bei einem Säuger.

2. Die Verwendung nach Anspruch 1, wobei die metastatischen Hirnstellen von Brustkrebs erbB-2 überexprimieren.

3. Die Verwendung, wie in Anspruch 1 oder Anspruch 2 beansprucht, wobei die Verbindung der Formel (I) oder ein pharmazeutisch verträgliches Salz davon Lapatinib-Ditosylat ist.

4. Die Verwendung, wie in Anspruch 1 oder Anspruch 2 beansprucht, wobei die Verbindung der Formel (I) oder ein pharmazeutisch verträgliches Salz davon Lapatinib-Ditosylat-Monohydrat ist.

5. Eine Verbindung der Formel (I) 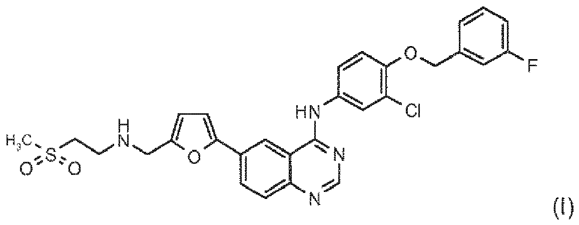 oder ein pharmazeutisch verträgliches Salz davon zur Verwendung bei der Behandlung von metastatischen Stellen von Brustkrebs im Gehirn bei einem Säuger.

6. Die Verbindung nach Anspruch 5 zur Verwendung wie in Anspruch 5 beansprucht, wobei die metastatischen Hirnstellen von Brustkrebs erbB-2 überexprimieren.

7. Die Verbindung nach Anspruch 5 zur Verwendung wie in Anspruch 5 oder Anspruch 6 beansprucht, wobei die Verbindung der Formel (I) oder ein pharmazeutisch verträgliches Salz davon Lapatinib-Ditosylat ist.

8. Die Verbindung nach Anspruch. 5 zur Verwerdung wie in Anspruch 5 oder Anspruch 6 beansprucht, wobei die Verbindung der Formel (I) oder ein pharmazeutisch verträgliches Salz davon Lapatinib-Ditosylat-Monohydrat ist.

## Revendications

1. Utilisation de quantités thérapeutiquement efficaces d'un composé de formule (I) 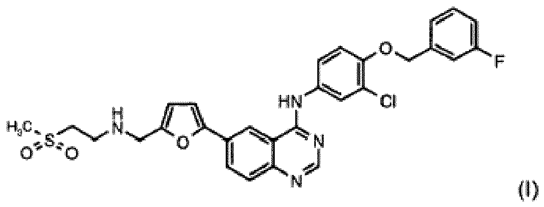 ou d'un de ses sels pharmaceutiquement acceptables dans la préparation d'un médicament pour le traitement chez un mammifère de sites métastatiques de cancer du sein dans le cerveau.

2. Utilisation suivant la revendication 1, dans laquelle les sites métastatiques cérébraux de cancer du sein présentent une surexpression de erbB-2

3. Utilisation suivant la revendication 1 ou la revendication 2, dans laquelle le composé de formule (I) ou un de ses sels pharmaceutiquement acceptables est le ditosylate de lapatinib.

4. Utilisation suivant la revendication 1 ou la revendication 2, dans laquelle le composé de formule (I) ou un de ses sels pharmaceutiquement acceptables est le monohydrate de ditosylate de lapatinib.

5. Composé de formule (I) 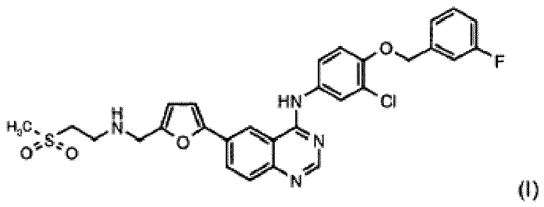 ou un de ses sels pharmaceutiquement acceptables, pour une utilisation dans le traitement chez un mammifère de sites métastatiques de cancer du sein dans le cerveau.

6. Composé de la revendication 5 pour une utilisation suivant la revendication 5, les sites métastatiques cérébraux de cancer du sein présentant une surexpression de erbB-2

7. Composé de la revendication 5 pour une utilisation suivant la revendication 5 ou la revendication 6, le composé de formule (I) ou un de ses sels pharmaceutiquement acceptables étant le ditosylate de lapatinib.

8. Composé de la revendication 5 pour une utilisation suivant la revendication 5 ou la revendication 6, le composé de formule (I) ou un de ses sels pharmaceutiquement acceptables étant le monohydrate de ditosylate de lapatinib.
